(19)

Europäisches Patentamt

European Patent Office

Office européen des brevets

(11) **EP 4 262 526 B1**

(12) **EUROPEAN PATENT SPECIFICATION**

(45) Date of publication and mention
of the grant of the patent:
**02.04.2025 Bulletin 2025/14**

(21) Application number: **21831027.4**

(22) Date of filing: **13.12.2021**

(51) International Patent Classification (IPC):
**A61B 5/00** (2006.01)     **A61B 5/021** (2006.01)
**A61B 5/024** (2006.01)

(52) Cooperative Patent Classification (CPC):
**A61B 5/7203; A61B 5/7239; A61B 5/7278;**
A61B 5/02108; A61B 5/02125; A61B 5/02416

(86) International application number:
**PCT/EP2021/085339**

(87) International publication number:
**WO 2022/128830 (23.06.2022 Gazette 2022/25)**

(54) **METHOD, APPARATUS AND COMPUTER PROGRAM PRODUCT FOR ANALYSING A PULSE WAVE SIGNAL**

VERFAHREN, VORRICHTUNG UND COMPUTERPROGRAMMPRODUKT ZUR ANALYSE EINES PULSWELLENSIGNALS

PROCÉDÉ, APPAREIL ET PRODUIT PROGRAMME INFORMATIQUE POUR L'ANALYSE D'UN SIGNAL D'ONDE D'IMPULSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2020 EP 20214268**

(43) Date of publication of application:
**25.10.2023 Bulletin 2023/43**

(73) Proprietor: **Koninklijke Philips N.V.**
**5656 AG Eindhoven (NL)**

(72) Inventor: **DERKX, Rene Martinus Maria**
**5656 AG Eindhoven (NL)**

(74) Representative: **Philips Intellectual Property & Standards**
**High Tech Campus 52**
**5656 AG Eindhoven (NL)**

(56) References cited:
**AU-A1- 2019 260 099     US-A1- 2016 228 069**

- **HANGSIK SHIN ET AL: "Feasibility study for the non-invasive blood pressure estimation based on ppg morphology: normotensive subject study", BIOMEDICAL ENGINEERING ONLINE, 10 January 2017 (2017-01-10), pages 1 - 14, XP055478171, Retrieved from the Internet <URL:https://www.ncbi.nlm.nih.gov/pmc/articles/PMC5234121/pdf/12938_2016_Article_302.pdf> [retrieved on 20180524], DOI: 10.1186/s12938-016-0302-y**

**Description**

FIELD OF THE INVENTION

This disclosure relates to pulse wave signals obtained from a subject, with the pulse wave signal comprising pulse wave measurements for a plurality of cardiac cycles of the subject. More particularly, this disclosure relates to a method, apparatus and a computer program product for analysing pulse wave signals.

BACKGROUND OF THE INVENTION

US2016228069A1 relates to determining vital signs of a subject.

Hangsik Shin et al: "Feasibility study for the non-invasive blood pressure estimation based on ppg morphology: normotensive subject study", Biomed. Eng. Online, 2017; 16: 10 highlights the PPG morphological analysis based on pressure-flow relationship and correlation analysis between blood pressure and derived parameter.

AU2019260099A1 relates to a method to estimate the blood pressure and the arterial stiffness based on photoplethysmographic (PPG) signals.

Blood pressure (BP) is an important indicator of health in a person/subject. In the US it is estimated about 30% of the adult population has high blood pressure. Hypertension is a common health problem which has no obvious outward symptoms. Blood pressure generally rises with aging and the risk of becoming hypertensive in later life is considerable. Persistent hypertension is one of the key risk factors for strokes, heart failure and increased mortality. The condition of a subject can be improved by lifestyle changes, healthy dietary choices and medication. Particularly for high risk patients, continuous 24-hour blood pressure monitoring is very important by means of systems which do not impede ordinary daily life activities. Continuous monitoring of blood pressure can also be useful for patients in a healthcare environment, such as a hospital e.g. in the operating room (OR) or the Intensive Care Unit (ICU). A low blood pressure can lead to a poor oxygenation of important organs and could result in organ damage. A too high blood pressure can cause bleeding which should be prevented especially during and after surgical procedures, specifically in brain surgeries.

In some cases, absolute measurements of blood pressure can be obtained, and in other cases relative measurements of blood pressure can be obtained, for example a measurement of a change in blood pressure. In particular, blood pressure can change over short time windows, e.g. of the order of a few minutes, and these changes can be relevant for further medical examination and possibly medical intervention.

There are a number of different techniques available for measuring blood pressure, and/or changes in blood pressure. Some of these techniques measure blood pressure itself, while other techniques measure other physiological characteristics of the subject and use these as surrogates for blood pressure, for example by relating changes or values of the physiological characteristic to changes or values of blood pressure. Some of the techniques for directly measuring blood pressure require invasive access to the arteries of the subject, or the use of bulky/inconvenient equipment such as inflatable cuffs. However, some of the physiological characteristics used as surrogates for blood pressure can be measured using simple and/or unobtrusive sensors applied to the body of the subject.

Tonometry uses an externally placed force or pressure sensor to measure arterial distension (i.e. a waveform representing the distension of the artery) as pressure is applied to the artery. Alternatively, one or more photoplethysmography (PPG) sensors can be placed on a part of the body to obtain one or more PPG signals that represent the changes in volume of the blood flow in the body part during a number of heart cycles (cardiac cycles). Both of these techniques obtain a pulse wave signal (PWS) from the subject that covers a number of cardiac cycles of the subject. This pulse waveform/signal can be analysed to determine one or more physiological characteristics that are used as surrogate blood pressure measurements.

One physiological characteristic that can be used as a surrogate blood pressure measurement is pulse wave velocity (PWV). When the heart beats, a pulse wave is generated through the blood of the aorta and the further arterial system. The speed of the pulse wave (called pulse wave velocity) is influenced by blood (fluid) properties and some arterial properties (like diameter and compliance). These blood properties and arterial properties are also influenced by blood pressure, and so changes in PWV can be linked to changes in blood pressure.

Some techniques for measuring PWV use a two-spot or dual-spot approach. This requires two sensors (e.g. PPG sensors) in order to capture two signals simultaneously. The signal from the first sensor is used to detect the onset of the pulse wave at a proximal location, e.g. close to the heart. The signal from the second signal is used to detect the arrival of the pulse wave at a distal location, e.g. at the femoral artery of the finger of the patient.

However, to minimise the inconvenience for the subject due to the measurement equipment, single-spot techniques for measuring PWV are being developed. These techniques make use of pulse wave reflections in the arterial tree. There is a direct pulse wave traveling from the aorta to, for example, the finger, and there is an indirect pulse wave that first travels from the aorta to the renal bifurcation and then travels from the renal bifurcation to the finger. In this way, the indirect (reflected) pulse wave arrives later at the finger location than the direct pulse wave. When the arrival times of the direct and indirect pulse waves are measured at the finger, a subtraction of these arrival times gives the time required to travel from the aortic arch to the renal bifurcation and back. With knowledge (or an approximation) of this extra travel distance of the reflected pulse wave, it is possible to estimate the pulse wave velocity of the reflected wave according to Equation (1) below:

$$\mathrm{PWV}_s = \frac{2 \cdot L_{hr}}{t_c - t_a} \triangleq \frac{2 \cdot L_{hr}}{\mathrm{PRT}},$$

(1)

where $L_{hr}$ is the distance between the aortic arch and the renal bifurcation and PRT is the so-called pulse reflection time, which is defined as the time between the start (up-flank) of the direct pulse wave until the time of the start (up-flank) of the indirect pulse wave. The times $t_c$ and $t_a$ required to compute the PRT can be determined via the so-called "acceleration waveform" of the PPG measurement, that is obtained via, e.g. double-differentiation of the PPG waveform with respect to time.

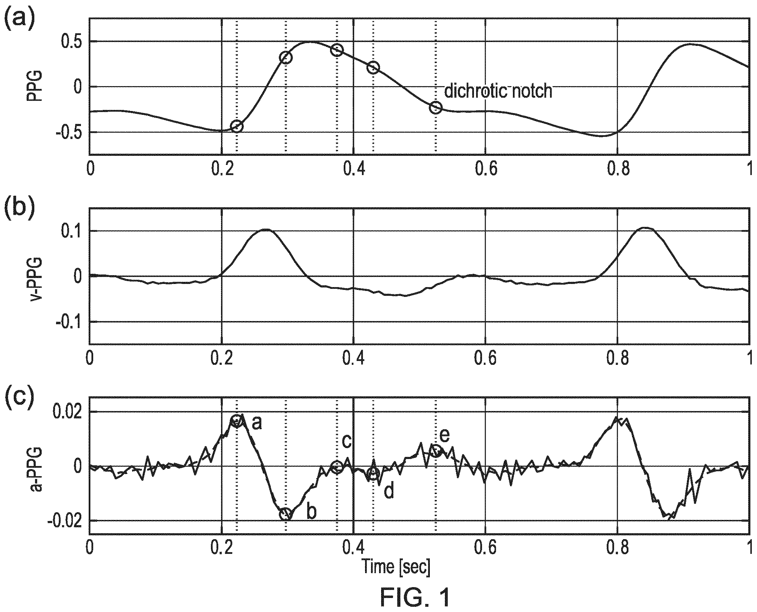

Typically, it is assumed that the acceleration waveform consists of five 'fiducial points' or 'reference points', as shown in Fig 1. Fig 1(a) shows an exemplary PPG signal covering a 1 second period with the dicrotic notch indicated. Fig. 1(b) shows a first derivative of the PGG signal of Fig. 1(a) with respect to time, and Fig. 1(c) shows a second derivative of the PGG signal of Fig. 1(a) with respect to time. The first derivative is denoted v-PPG and the second derivative (acceleration waveform) is denoted a-PPG. The five reference points are shown in the acceleration waveform, Fig 1(c). The a point marks the start of the direct pulse wave and the b point marks the end of the direct pulse wave. The e point marks the end of the systolic period (aortic valve closure), and for the purposes of this disclosure it is assumed that the c point marks the start of the reflected wave and the d point marks the end of the reflected wave.

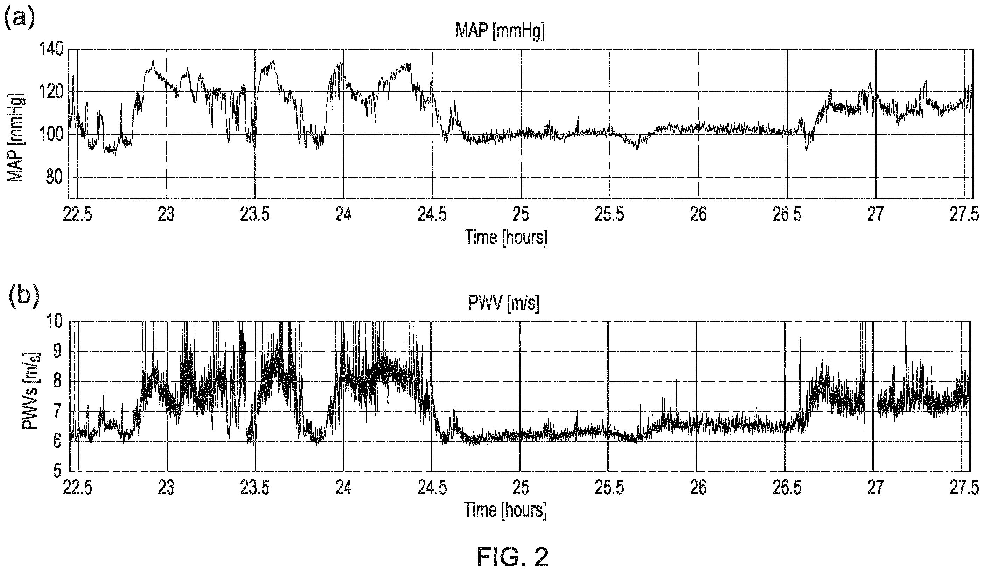

Fig. 2 shows an example of a single-spot PPG measurement and PWV derivation according to Equation (1). The graph in Fig. 2(a) shows the mean arterial pressure (MAP) in mmHg over a period of 5 hours of an ICU patient. The graph in Fig. 2(b) shows PWV in m/s calculated from a PPG signal according to Equation (1), with an estimation of $2L_{hr}$ (twice the heart to the renal bifurcation distance) as 75 cm.

It can be seen in Fig. 2 that there is a strong positive correlation between the MAP and the pulse wave velocity from the single-spot measurement. However, the robustness of the pulse wave velocity measurement can be questioned since there are a lot of outliers in the pulse wave velocity values, particularly when compared to pulse wave velocity derived from a two-spot measurement approach.

SUMMARY OF THE INVENTION

The invention is defined by the independent claims. The dependent claims define advantageous embodiments.

Part of the problem with the single-spot pulse wave velocity measurement is that the fiducial points of the reflected pulse waves are not always easy to detect. This can be seen from the a-PPG plot in Fig. 1(c). Computing a second derivative of this PPG signal leads to a very poor signal because of the quantization and noise in the original PPG signal. Signal smoothing (either temporal or over multiple cardiac cycles) can be applied in order to obtain improved fiducial point detections. After such smoothing, it becomes possible to robustly detect the fiducial points. However, for a good detection of the c and d points (which relate to the reflected wave) quite substantial smoothing needs to be applied before the fiducial points can be robustly detected. Too much temporal smoothing leads to loss in the high-frequency fiducial points, whereas too much averaging over multiple cardiac cycles gives problems under time varying conditions).

Therefore, there is a need for improvements in the averaging of cardiac cycle waveforms.

The techniques described herein apply averaging over multiple cardiac cycles to reduce or remove the noise in the resulting average to enable an improved analysis of reflected pulse waves and/or other characteristics of the cardiac cycle waveform. As noted above, analysis of reflected pulse waves can be used as a surrogate blood pressure measurement, but it will be appreciated that analysis of an averaged cardiac cycle waveform can be used for monitoring other aspects of the health of the subject, e.g. trends of arterial compliance of a patient during a full hospital stay.

According to a first specific aspect, there is provided a computer-implemented method for analysing a pulse wave signal (PWS) obtained from a subject. The PWS comprises pulse wave measurements for a plurality of cardiac cycles of the subject during a first time period. The method comprises (i) analysing the PWS to identify a plurality of cardiac cycles and a respective reference point for each identified cardiac cycle; (ii) determining 2PWS as a second derivative with respect to time of the PWS; (iii) determining a normalised 2PWS by, for each part of the 2PWS corresponding to a respective identified cardiac cycle, normalising said part of the 2PWS with respect to the amplitude of the 2PWS at the identified reference point for said cardiac cycle; (iv) for a first lag time value, determining an n-th order polynomial fit for a first set of values of the normalised 2PWS, wherein the first set of values of the normalised 2PWS comprises the values of the normalised 2PWS occurring the first lag time value from the reference point of each identified cardiac cycle, wherein n is equal to or greater than 1; (v) performing one or more further iterations of step (iv) for one or more further lag time values to determine respective further n-th order polynomial fits for respective sets of values of the normalised 2PWS, wherein a respective set of values of the normalised 2PWS comprises the values of the normalised 2PWS that occur the respective further lag time value from the reference point of each identified cardiac cycle; and (vi) forming a first average cardiac cycle waveform for a first time point in the first time period, wherein the first average cardiac cycle waveform is formed from values of the plurality of n-th order polynomial fits at the first time point. Therefore, this aspect provides an improved average cardiac cycle waveform that takes into account a trend in the PWS over the first time period, and that allows for improved analysis of a PWS obtained using, for example, a single-spot measurement technique.

In some embodiments, the method further comprises forming a second average cardiac cycle waveform for a second time point in the first time period, wherein the second average cardiac cycle waveform is formed from values of the plurality of n-th order polynomial fits at the second time point.

In these embodiments, the method can further comprise comparing the first average cardiac cycle waveform and the second average cardiac cycle waveform to determine a change in the average cardiac cycle waveform between the first time point and the second time point. These embodiments provide that changes in the average cardiac cycle waveform over the first time period can be evaluated, for example to evaluate how a property related to the cardiac cycle has changed.

In these embodiments, the method can further comprise determining a measure of the blood pressure of the subject, or a measure of a change in blood pressure of the subject, from the first average cardiac cycle waveform and the second average cardiac cycle waveform.

In some embodiments, the method further comprises processing the first average cardiac cycle waveform to determine a measure of the blood pressure of the subject.

In some embodiments, each of the first lag time value and the one or more further lag time values are equal to or less than a duration of a cardiac cycle of the subject.

In some embodiments, the reference point for each identified cardiac cycle is an onset of a pulse wave of the subject. This reference point is useful as it is relatively easy to detect in first or second derivative of the PWS with respect to time.

In some embodiments, step (ii) is performed prior to, or as part of, step (i), and step (i) can comprise identifying the plurality of cardiac cycles and the respective reference point for each identified cardiac cycle as local maxima in the 2PWS.

In alternative embodiments, step (ii) is performed prior to, or as part of, step (i), and step (i) can comprise detecting peaks in a first derivative with respect to time of the PWS (1PWS); and identifying the plurality of cardiac cycles and the respective reference point for each identified cardiac cycle as local maxima in the 2PWS within respective search windows defined by the detected peaks in the 1PWS.

In some embodiments, n is 1. In other embodiments, n is 2.

In some embodiments, the PWS is a photoplethysmogram (PPG) signal.

According to a second aspect, there is provided an apparatus for analysing a pulse wave signal (PWS) obtained from a subject. The PWS comprises pulse wave measurements for a plurality of cardiac cycles of the subject during a first time period. The apparatus is configured to: (i) analyse the PWS to identify a plurality of cardiac cycles and a respective reference point for each identified cardiac cycle; (ii) determine 2PWS as a second derivative with respect to time of the PWS; (iii) determine a normalised 2PWS by, for each part of the 2PWS corresponding to a respective identified cardiac cycle, normalising said part of the 2PWS with respect to the amplitude of the 2PWS at the identified reference point for said cardiac cycle; (iv) for a first lag time value, determine an n-th order polynomial fit for a first set of values of the normalised 2PWS, wherein the first set of values of the normalised 2PWS comprises the values of the normalised 2PWS occurring the first lag time value from the reference point of each identified cardiac cycle, wherein n is equal to or greater than 1; (v) perform one or more further iterations of operation (iv) for one or more further lag time values to determine respective further n-th order polynomial fits for respective sets of values of the normalised 2PWS, wherein a respective set of values of the normalised 2PWS comprises the values of the normalised 2PWS that occur the respective further lag time value from the reference point of each identified cardiac cycle; and (vi) form a first average cardiac cycle waveform for a first time point in the first time period, wherein the first average cardiac cycle waveform is formed from values of the plurality of n-th order polynomial fits at the first time point. Therefore, this aspect provides an improved average cardiac cycle waveform that takes into account a trend in the PWS over the first time period, and that allows for improved analysis of a PWS obtained using, for example, a single-spot measurement technique.

In some embodiments, the apparatus is further configured to form a second average cardiac cycle waveform for a second time point in the first time period, wherein the second average cardiac cycle waveform is formed from values of the plurality of n-th order polynomial fits at the second time point.

In these embodiments, the apparatus can be further configured to compare the first average cardiac cycle waveform and the second average cardiac cycle waveform to determine a change in the average cardiac cycle waveform between the first time point and the second time point. These embodiments provide that changes in the average cardiac cycle waveform over the first time period can be evaluated, for example to evaluate how a property related to the cardiac cycle has changed.

In these embodiments, the apparatus can be further configured to determine a measure of the blood pressure of the subject, or a measure of a change in blood pressure of the subject, from the first average cardiac cycle waveform and the second average cardiac cycle waveform.

In some embodiments, the apparatus can be further configured to process the first average cardiac cycle waveform to determine a measure of the blood pressure of the subject.

In some embodiments, each of the first lag time value and the one or more further lag time values are equal to or less than a duration of a cardiac cycle of the subject.

In some embodiments, the reference point for each identified cardiac cycle is an onset of a pulse wave of the subject. This reference point is useful as it is relatively easy to detect in first or second derivative of the PWS with respect to time.

In some embodiments, operation (ii) is performed prior to, or as part of, operation (i), and operation (i) can comprise identifying the plurality of cardiac cycles and the respective reference point for each identified cardiac cycle as local maxima in the 2PWS.

In alternative embodiments, operation (ii) is performed prior to, or as part of, operation (i), and operation (i) can comprise detecting peaks in a first derivative with respect to time of the PWS (1PWS); and identifying the plurality of cardiac cycles and the respective reference point for each identified cardiac cycle as local maxima in the 2PWS within respective search windows defined by the detected peaks in the 1PWS.

In some embodiments, n is 1. In other embodiments, n is 2.

In some embodiments, the PWS is a photoplethysmogram (PPG) signal.

In some embodiments, the apparatus further comprises a pulse wave sensor for obtaining the PWS from the subject. In alternative embodiments, the apparatus is configured to receive the PWS from a pulse wave sensor.

According to a third aspect, there is provided a computer program product comprising a computer readable medium having computer readable code embodied therein, the computer readable code being configured such that, on execution by a suitable computer or processor, the computer or processor is caused to perform the method according to the first aspect or any embodiment thereof.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1(a) shows a 1-second segment of a PPG signal, and Figs 1(b) and 1(c) show the first and second derivatives of the PPG signal respectively;
Fig. 2(a) shows a measurement of mean arterial pressure over a 5-hour time period, and Fig. 2(b) shows an example of a single-spot PPG measurement and PWV derivation according to Equation (1) for the same time period;
Fig. 3 is a block diagram of an apparatus according to various exemplary embodiments;
Fig. 4 is a flow chart illustrating a method for analysing a pulse wave signal obtained from a subject according to various embodiments;
Fig. 5 shows a 1-second segment of a PPG signal, and Figs 5(b) and 5(c) show the first and second derivatives of the PPG signal respectively;
Fig. 6(a) shows a graph of arterial blood pressure in a 1-minute time window and Fig. 6(b) shows a graph of a second derivative of a pulse wave signal during the 60-second time window;
Fig. 7 is a graph showing a plurality of waveforms identified in the second derivative of the pulse wave signal overlaid with each other;
Fig. 8 is a graph showing a 0-th order average of the plurality of waveforms shown in Fig. 7;
Fig. 9 is a graph showing values from the plurality of waveforms identified in the second derivative of the pulse wave signal at a particular lag time value from a common reference point, and three n-th order polynomial fits for the displayed values;
Fig. 10 is a graph showing 1st order average waveforms for several time points during the 60-second time window; and
Fig. 11 is a functional block diagram illustrating various operations in analysing a pulse wave signal according to various embodiments.

DETAILED DESCRIPTION OF EMBODIMENTS

The techniques described herein apply averaging over multiple cardiac cycles to reduce or remove the noise in the resulting average to enable an improved analysis of reflected pulse waves and/or other characteristics of the cardiac cycle waveform. Analysis of reflected pulse waves can be used as a surrogate blood pressure measurement, but it will be appreciated that analysis of an averaged cardiac cycle waveform can provide information about other aspects of the health of the subject. The described techniques are particularly useful for the so-called single-spot measurement techniques where a single sensor is applied to a subject.

For a pulse wave signal (PWS) that includes information about pulse changes/pulse waves at a measurement point on a body of a subject, reference points for each of the cardiac cycles to be smoothed are identified in the PWS. The PWS can be, e.g., a PPG signal or a pulse wave signal obtained using tonometry. The reference point to be identified preferably relates to the initial up-flank of the pulse wave, which should be free of influences of reflections and hence should be a stable reference point (i.e. not dependent on blood pressure changes). However it will be appreciated that a different reference point can be used if desired. Next, the times and amplitudes of the various occurrences of the reference points are used in order to compute an average cardiac cycle waveform. Normal averaging of all cardiac cycle waveforms across the time window does not allow for time-varying circumstances. Such normal averaging is described in WO 2015/044010. The averaging technique described herein extends the averaging to allow for (linear) variation in time for each lag in the averaging procedure (where the lag, or lag time, is a time relative to the identified reference point for each cardiac cycle, e.g. the time relative to the identified *a* reference point for each cardiac cycle).

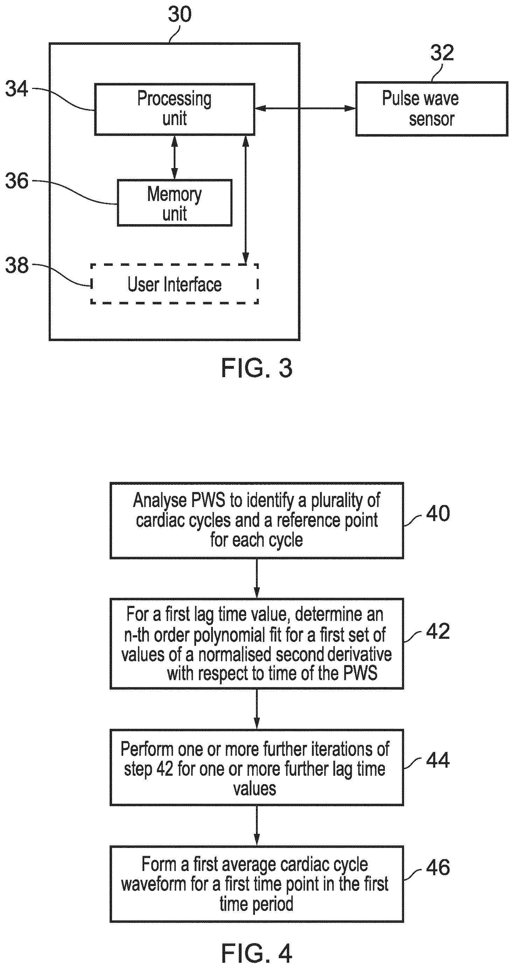

Fig. 3 is a block diagram of an apparatus 30 for analysing a PWS according to various embodiments of the techniques described herein. A pulse wave sensor 32 is shown in Fig. 3 that is used to measure pressure waves at a single point on the body of a subject and to output a PWS. The pulse wave sensor 32 can be a PPG sensor, a tonometry-based sensor or a hydraulic sensor pad that can be applied with some application pressure to the (upper) arm of the subject and using a pressure sensor of any type, e.g. the MPXV6115 Series Integrated Silicon Pressure Sensor from NXP Semiconductors. In some embodiments, the pulse wave sensor 32 can be part of, or integral with, the apparatus 30. In other embodiments, the apparatus 30 can be connected to the pulse wave sensor 32, either directly (e.g. wired) or indirectly (e.g. using a wireless communication technology such as Bluetooth, WiFi, a cellular communication protocol, etc.). In alternative embodiments, the apparatus 30 may not be connected to the pulse wave sensor 32, and instead the apparatus 30 can obtain the PWS from another device or apparatus, such as a server or database.

As is known, a PPG sensor 32 can be placed on the body of the subject, for example on an arm, leg, earlobe, finger, etc., can provide an output signal (a 'PPG signal') that is related to the volume of blood passing through that part of the body. The volume of blood passing through that part of the body is related to the pressure of the blood in that part of the body. A PPG sensor 32 typically comprises a light sensor, and one or more light sources. The PPG signal output by the PPG sensor 32 may be a raw measurement signal from the light sensor (e.g. the PPG signal can be a signal representing light intensity over time). Alternatively, the PPG sensor 32 may perform some pre-processing of the light intensity signal, for example to reduce noise and/or compensate for motion artefacts, but it will be appreciated that this pre-processing is not required for the implementation of the techniques described herein.

The apparatus 30 may be in the form of, or be part of, a computing device, such as a server, desktop computer, laptop, tablet computer, smartphone, smartwatch, etc., or a type of device typically found in a clinical environment, such as a patient monitoring device (e.g. a monitoring device located at the bedside of a patient in a clinical environment) that is used to monitor (and optionally display) various physiological characteristics of a subject/patient.

The apparatus 30 includes a processing unit 34 that controls the operation of the apparatus 30 and that can be configured to execute or perform the methods described herein to analyse the PWS. The processing unit 34 can be implemented in numerous ways, with software and/or hardware, to perform the various functions described herein. The processing unit 34 may comprise one or more microprocessors or digital signal processors (DSPs) that may be programmed using software or computer program code to perform the required functions and/or to control components of the processing unit 34 to effect the required functions. The processing unit 34 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The processing unit 34 is connected to a memory unit 36 that can store data, information and/or signals for use by the processing unit 34 in controlling the operation of the apparatus 30 and/or in executing or performing the methods described herein. In some implementations the memory unit 36 stores computer-readable code that can be executed by the processing unit 34 so that the processing unit 34 performs one or more functions, including the methods described herein. In particular embodiments, the program code can be in the form of an application for a smartwatch, smartphone, tablet, laptop or computer. The memory unit 36 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit 36 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In some embodiments, the apparatus 30 comprises a user interface 38 that includes one or more components that enables a user of apparatus 30 to input information, data and/or commands into the apparatus 30, and/or enables the apparatus 30 to output information or data to the user of the apparatus 30. Information that can be output by the user interface 38 can include an indication or illustration of an averaged cardiac cycle waveform for one or more time points, and/or information derived from an averaged cardiac cycle waveform. The user interface 38 can comprise any suitable input component(s), including but not limited to a keyboard, keypad, one or more buttons, switches or dials, a mouse, a track pad, a touchscreen, a stylus, a camera, a microphone, etc., and/or the user interface 38 can comprise any suitable output component(s), including but not limited to a display screen, one or more lights or light elements, one or more loudspeakers, a vibrating element, etc.

It will be appreciated that a practical implementation of an apparatus 30 may include additional components to those shown in Fig. 3. For example the apparatus 30 may also include a power supply, such as a battery, or components for enabling the apparatus 30 to be connected to a mains power supply. The apparatus 30 may also include interface circuitry for enabling a data connection to and/or data exchange with other devices, including the pulse wave sensor 32 (in embodiments where the pulse wave sensor 32 is separate from the apparatus 30), servers, databases, user devices and/or other sensors.

The flow chart in Fig. 4 shows an exemplary method for analysing a PWS obtained from a subject according to various embodiments. In some embodiments, the processing unit 34 in the apparatus 30 can be configured to implement the method in Fig 4. In other embodiments computer readable code can be provided that causes a computer or the processing unit 34 to perform the method of Fig. 4 when the computer or processing unit 34 executes the code.

The PWS is received from a pulse wave sensor 32 located at a single measurement point on the subject and the PWS represents pulse wave measurements for a plurality of cardiac cycles (i.e. heart beats) of the subject. The PWS is obtained with a sampling rate $F_s$. The method in Fig. 4 is described below with reference to a PWS in the form of a PPG signal, but it will be appreciated that the method can be applied to other forms of PWS. In some embodiments, the method in Fig. 4 can be performed periodically or continuously on a PWS as it is received or measured from the subject. In some embodiments, the method of Fig. 4 can operate on a windowed portion of a longer PWS, for example a 1-minute window of a PWS covering a time period of 1 hour. However, it should be appreciated that the method can be applied to a PWS having any desired length that covers any number of cardiac cycles. In the following description, references to operations or steps being performed on the PWS relates to performing those operations or steps on the part of the PWS of interest, e.g. a part corresponding to a 1-minute time period.

In a first step of the method, step 40, the PWS is analysed to identify cardiac cycles, and a respective reference point is identified for each cardiac cycle. The reference point is a point in each cardiac cycle that can be used in subsequent steps to 'align' the cardiac cycles and enable an average to be determined.

As described above with reference to Fig. 1, each cardiac cycle in an 'acceleration waveform' (the second derivative of the PWS with respect to time) can be considered to comprise five 'fiducial points' or 'reference points'. The *a* reference point marks the start of the direct pulse wave, which is the onset of the pulse wave (i.e. the pulse of blood caused by the beat of the heart) and the *b* point marks the end of the direct pulse wave. The *e* point marks the end of the systolic period (aortic valve closure), and for the purposes of this disclosure it is assumed that the c point marks the start of the reflected wave and the *d* point marks the end of the reflected wave. Preferably, in step 40 the reference point for each cardiac cycle is the onset of the pulse wave (the direct part of the pulse wave) which can be seen as the start of the systole phase - i.e. reference point *a*. However, it will be appreciated that in other embodiments, other ones of the reference points can be identified in step 40, or indeed a different reference point to reference points *a* to *e* shown in Fig. 1(c).

In the following, the signal corresponding to the first derivative of the PWS with respect to time is denoted '1PWS' and the signal corresponding to the second derivative of the PWS with respect to time is denoted '2PWS'. When described with reference to the specific example of a PPG signal, the 1PWS is also referred to as a 'velocity waveform' (v-PPG) and the 2PWS is also referred to as an 'acceleration waveform' (a-PPG).

Some embodiments of step 40 provide for the detection of the onsets of the pulse wave by detecting the local maxima in each cardiac cycle represented in the 2PWS. However, it can be seen in Fig. 1(c) that a double-differentiated PPG signal can be of low quality because of noise or quantization in the original PPG signal.

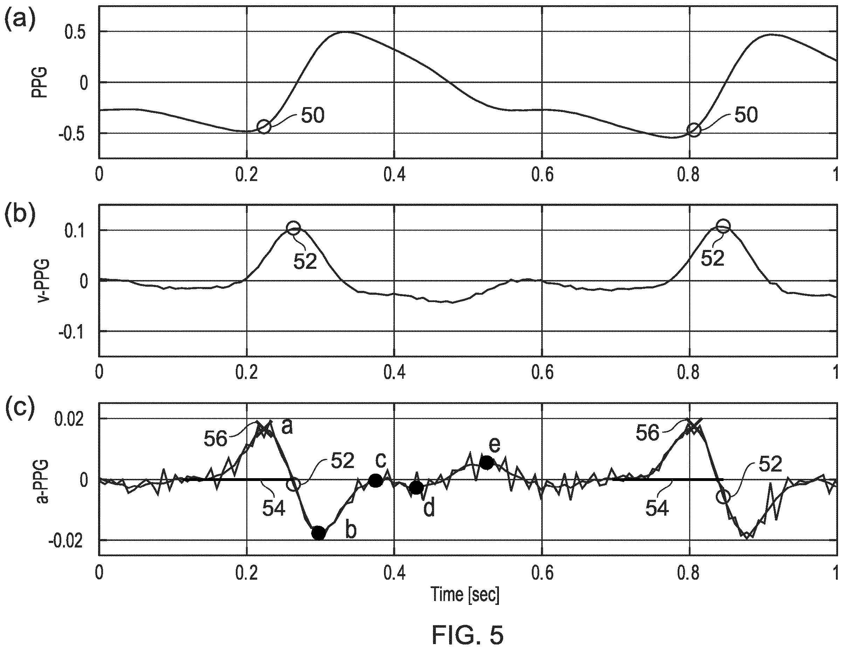

Therefore, in a more preferred embodiment, the onset of the pulse wave is detected using a two stage process. Fig. 5(a) shows the same 1-second segment of the PPG signal shown in Fig. 1(a). Fig. 5(b) shows the first derivative of the PPG signal with respect to time (v-PPG), and Fig. 5(c) shows the second derivative of the PPG signal with respect to time (a-PPG). In Fig. 5(a), the onset of the pulse wave for the two cardiac cycles is indicated by the points labelled 50, and the aim of this embodiment is to identify these points. In the first stage, peak detection is performed on the first derivative of the PWS with respect to time (1PWS). It can be seen in Fig. 5(b) that the peaks in the v-PPG signal will correspond roughly to the steepest flank in the original PPG waveform (shown in Fig. 5(a)). The peaks detected in the v-PPG signal are labelled 52.

After detecting the maximum velocity peaks 52 in the 1PWS, then in the second stage narrowed (local) search windows are applied to the 2PWS (e.g. the a-PPG shown in Fig. 5(c)) based on the timing of each of the maximum velocity peaks 52, and the maximum peaks in those narrowed search windows on the 2PWS are detected. The narrowed search window can have a duration of 30-100 ms. These maximum peaks correspond to the desired *a* reference points, i.e. the onset of the pulse wave corresponding to the up-flank in the PWS signal. In the a-PPG waveform shown in Fig 5(c), the narrowed search windows are indicated by the horizontal lines 54 that end at the timing of the detected velocity peaks 52. The maximum peaks identified in those narrowed search windows on the a-PPG waveform are labelled 56, and correspond to the desired *a* reference points.

Since the 2PWS can be noisy in case of poorly quantized signals, it can be beneficial to perform some smoothing prior to detection of the peaks in the 1PWS. This smoothing can be applied to the PWS before differentiation, or applied to the 1PWS before peak detection is performed. In some embodiments the smoothing can be achieved using by filtering, e.g. Savitzky-Golay filtering. The result of the smoothing process and differentiating a smoothed PWS/1PWS to determine the 2PWS can be seen by the smoothed line in Fig. 5(c).

In Fig. 5(c) the maxima/minima of the *b, c, d* and *e* waves are also shown for the first cardiac cycle. It can be seen that the *c* and *d* waves are very small (even in this best case situation) and will typically be very difficult to detect robustly. This illustrates the preference for the detection of the *a* reference point, but as noted above it would be possible to target the detection of other reference points in step 40. In the following, the detected *a* reference points for multiple cardiac cycles are used to perform an averaging of multiple cardiac cycles in order to determine an average cardiac cycle waveform.

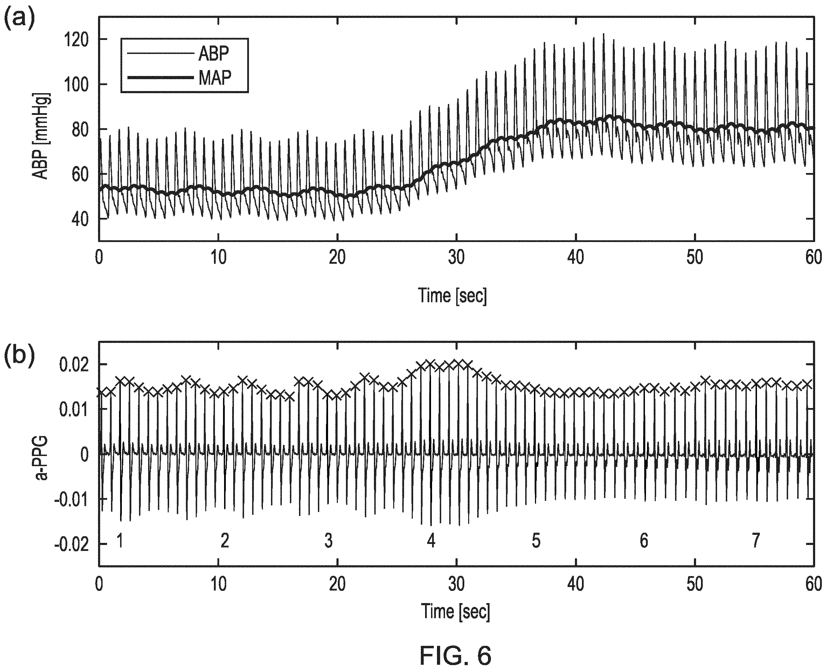

Next, in steps 42, 44 and 46, an averaging technique is applied to the PWS to determine an average cardiac cycle waveform for one or more time points $Y$ in the time period covered by the PWS. Steps 42, 44 and 46 are described below with reference to the exemplary 2PWS (in the form of an a-PPG) shown in Fig. 6(b). The 2PWS in Fig. 6(b) is derived from a PPG signal covering a 1-minute time period for a particular subject. This 1-minute time period covers 74 cardiac cycles of the subject. The respective reference points identified in step 40 for each cardiac cycle in the a-PPG waveform are marked in Fig. 6(b). Fig. 6(a) shows measurements of the arterial blood pressure (ABP - the thin line) and mean arterial pressure (MAP - the thicker line) for the same subject and same time period that the a-PPG signal in Fig. 6(b) relates to. The ABP measurements are shown in Fig. 6(a) to provide context for the a-PPG waveform in Fig. 6(b), and it will be appreciated that an ABP measurement will typically not be available for a subject for which a single-spot PWS measurement is being obtained. It can be seen in Fig. 6(a) that there is an increase in the mean arterial pressure of the order of 20 mmHg between 20 and 40 seconds. In the a-PPG waveform (Fig. 6(b)) there is some visible change at around 30 seconds (e.g. high respiratory modulation of the *a* reference points from 0-30 seconds and low respiratory modulation of the *a* reference points from 30-60 seconds), and the averaging technique described herein can be used to identify and/or analyse the changes in the morphology of the a-PPG waveform over and/or throughout the 60-second window.

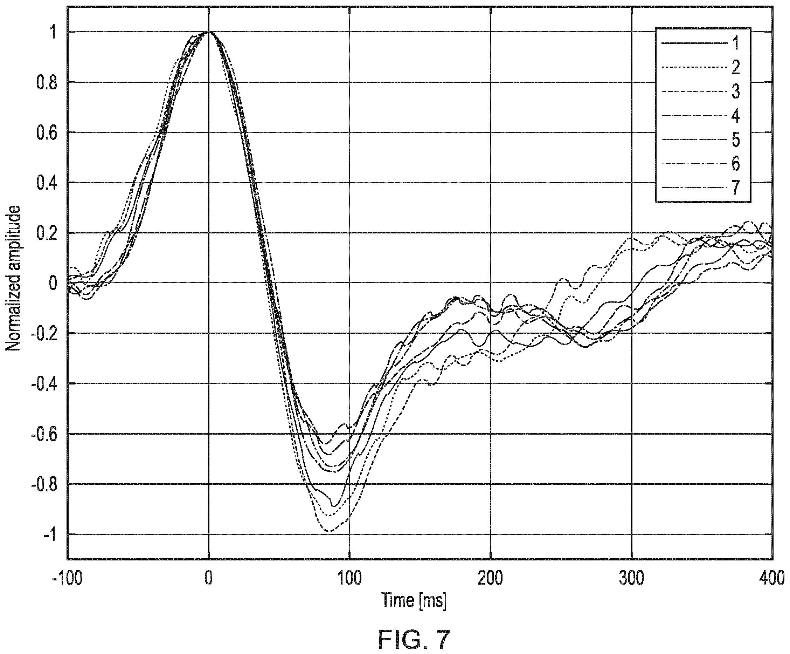

To show how the morphology of the a-PPG changes over the 60-second duration, Fig. 7 is a graph showing seven cardiac cycles from the a-PPG waveform of Fig. 6(b) overlaid with each other. The seven cardiac cycles included in Fig. 7 are labelled 1 to 7 in Fig. 6(b), and they are relatively evenly spaced through the 1-minute time window. It should be noted that these 7 cardiac cycles have been arbitrarily chosen from the cardiac cycles identified in Fig. 6(b) simply to provide a representation of how the cardiac cycle can change. In Fig. 7 the a-PPG waveforms for the 7 selected cardiac cycles are overlaid with the *a* reference points of each a-PPG waveform aligned, and the respective a-PPG waveforms are normalised so that the amplitude of each a-PPG waveform at the aligned *a* reference points are the same (amplitude = 1), and at other times the a-PPG waveforms have respective amplitudes that are usually in the range of -1 to 1. The time measured from the aligned reference points is referred to as the "lag time" or "lag time value", and is denoted $T_{\Delta k}$, with the aligned reference points corresponding to lag time $T_{\Delta k} = 0$ seconds. Each of the 7 waveforms shown in Fig. 7 are referred to as 'normalised' a-PPG waveforms herein, i.e. they are normalised around a common part of the cardiac cycle (e.g. reference point 'a' of each cardiac cycle in this example), with the amplitudes of the a-PPG waveforms matched at that reference point.

It can be seen in Fig. 7 that the complete morphology (after the *a* reference point) changes over time because of the blood pressure change. It can also be seen that it is very difficult to identify or analyse the waveforms separately, in part due to the noise in the second-derivative computation.

Since the pulse wave morphology can be constantly changing, as shown in the example of Fig. 7, simply applying an averaging to the multiple waveforms would lose the high-frequency information that is relevant for the analysis of the reflected pulse wave. Therefore, an averaging method is required where a linear change can be taken into account. The averaging technique disclosed herein is based on the technique in WO 2015/044010 with an extension to accommodate the linear change.

For describing the algorithm hereafter, a vector $\underline{x}$ is defined as the a-PPG data in a time window with a duration of $N_x/F_s$ seconds. The reference points (which in the following worked example are the peak locations, the *a* reference points) of the waveform selection x are listed as a vector $\underline{p}$ that has a length $N_p$, where $N_p$ is the number of cardiac cycles/identified reference points. In the example of Figs. 6 and 7, $N_p$ is 74. The waveform values at the detected peak locations are denoted

by $\underline{x}_{(p)_j}$ where $j = 0, ..., N_p-1$ is the index of the peak in each heart cycle. The values from the peak locations will be denoted in short format later on as

$$\underline{x}_{(\underline{p})_j} \triangleq (\underline{x}_p)_j.$$

(2)

where $j = 0,..., N_p-1$ is the index of the peak.

The next step in the averaging process is to analyse the $N_p$ neighbouring samples (towards the left and right with respect to the initial peaks (the *a* reference points) and compute the average change (decrease) compared to the initial peak level. Similar to the peaks, the neighbouring locations with respect to the peak locations are defined in short format as:

$$\underline{x}_{(\underline{p})_j + \Delta_k} \triangleq (\underline{x}_{p+\Delta_k})_j$$

(3)

where $\Delta_k$ is the lag index with respect to the peak locations, which can be either positive or negative valued. The lag index $\Delta_k$ relates to the lag time $T_{\Delta k}$ via:

$$\Delta_k = T_{\Delta k} \cdot F_s$$

(4)

For all neighbouring locations with respect to the peak locations which are inside the 1-minute window, the average level change (drop) is computed as:

$$\psi_{\Delta_k} = \frac{1}{N} \sum_{\substack{j=0 \\ 0 \le [(\underline{p})_j + \Delta_k] < N_x}}^{N_p-1} \frac{(\underline{x}_p)_j - (\underline{x}_{p+\Delta_k})_j}{(\underline{x}_p)_j},$$

(5)

where $N$ equals the number of averaged values that are in the region $[0...N_x-1]$.

This means that $N$ in the averaging procedure is not necessarily equal to the number of peaks $N_p$, and will be 1 or 2 smaller depending on whether some of the values $(\underline{p})_j + \Delta_k$ are outside the window with length $N_x$.

The computation of the model is done for several negative and positive values of $\Delta_k$, called iterations or repetitions. All the average values in the average model can be independently computed. Since only the average model values in the systole phase of the heart-cycle are of interest, bounds for the negative and positive values of $\Delta_k$ can be applied. For the negative values of $\Delta_k$ only negative values that are part of the start of the systole phase can be included. Since the peak location is very close to this start of the systole, the negative values of $\Delta_k$ can be limited to be equivalent to e.g. -0.1 seconds. For the positive values of $\Delta_k$, lag times that are part of the remainder of the systole are included. Typical maximum positive values for $\Delta_k$ can be chosen to be equivalent to lag times $T_{\Delta k}$ of e.g. 0.4 seconds. The average normalised waveform $\underline{w}$ can now be computed for all lag indices $\Delta_k$ as:

$$(\underline{w})_{\Delta_k} = 1 - \psi_{\Delta_k},$$

(6)

with $\psi_{\Delta_k}$ as computed by Equation (5).

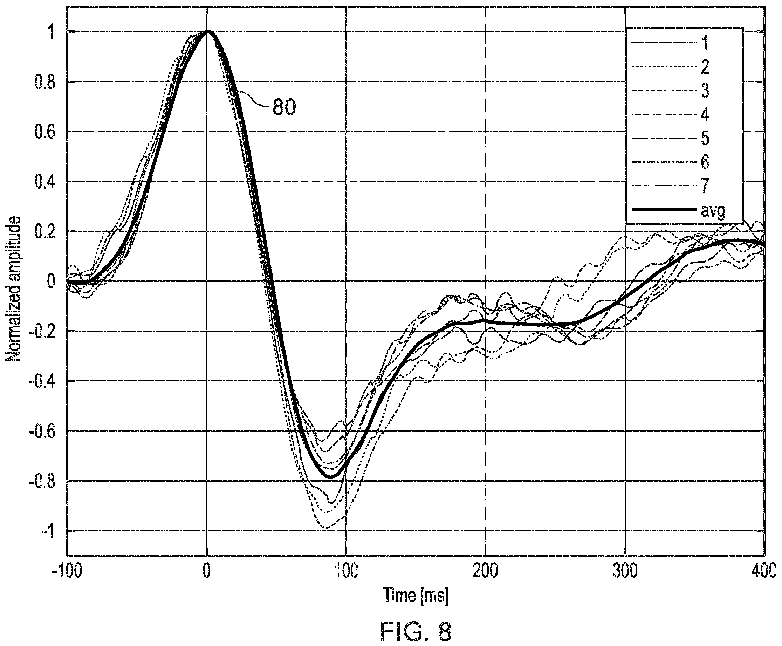

Fig. 8 shows the average normalised waveform (the thicker line 80) overlaid on the seven cardiac cycles from the normalised a-PPG waveforms shown in Fig. 7. In effect, the average normalised waveform 80 from the above process is obtained by taking the average (mean) of each normalised cardiac cycle at each lag time value ($T_{\Delta k}$). For example, the value (i.e. normalised amplitude) of the average waveform 80 at lag time $T_{\Delta k} = +100$ms is given by the average (mean) of the values of the individual normalised a-PPG waveforms at lag time $T_{\Delta k} = +100$ms (i.e. normalised in time and amplitude relative to the identified *a* reference point for each cardiac cycle). This average waveform 80 can also be described as a 0-th order polynomial fit for the normalised a-PPG cardiac cycles.

However, as shown in Fig. 8, this average over the full 1-minute window is not a good fit for all of the normalised cardiac cycles in the full 1-minute window since the morphology of the waveform is varying over time.

Therefore, the above averaging procedure is generalised to accommodate time-varying situations by not simply computing the 0-th order average of the $N_p$ values at lag time $T_{\Delta k}$, but a $1^{st}$ order or higher polynomial fit of the $N_p$ values at lag time $T_{\Delta k}$. Since this polynomial fit, having order *n* (where *n is* equal to or greater than 1) will have (lag) time on the x-axis and the level drop values on the y-axis, the averaged (or curve fitted) level drop will also have a dependency on time for polynomial orders larger than 0. For each of the lag times $\Delta_k$, $m$ $(m \le n)$ polynomial curve fit coefficients can be computed. These coefficients $a_0,...,a_m$ can be computed in such a way to obtain a minimisation in the least-squares sense:

$$\arg \min_{a_0,...,a_m} \sum_{\substack{j=0 \\ 0 \le [(\underline{p})_j + \Delta_k] < N_x}}^{N_p - 1} \left( \frac{(\underline{x}_p)_j - (\underline{x}_{p+\Delta_k})_j}{(\underline{x}_p)_j} - M_{j,\Delta_k}^n \right)^2 ,$$ (7)

where $M_{j,\Delta_k}^n$ is the linear fitting model:

$$M_{j,\Delta_k}^n = a_0(\Delta_k) + a_1(\Delta_k) \cdot [(\underline{p})_j + \Delta_k] + ... + a_m(\Delta_k) \cdot [(\underline{p})_j + \Delta_k]^n$$ (8)

Next, based on the polynomial coefficients $a_0(\Delta_k), ..., a_m(\Delta_k)$, the average level change (drop) can be computed directly via the linear fitting model $M_{j,\Delta_k}^n$ as:

$$\psi_{j,\Delta_k}^n = M_{j,\Delta_k}^n .$$ (9)

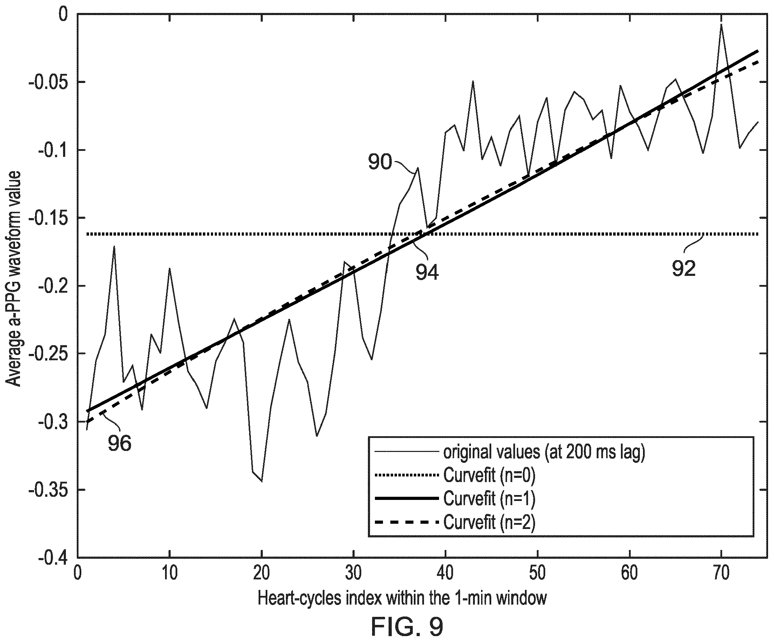

It can be seen that for $n = 0$, we obtain the average level change (drop) as given by Equation (5) and via using Equation (6) we get the average fitting model as described in WO 2015/044010 and shown in Fig. 8, which will not depend on the actual location within the time window (e.g. the 1-minute window in this example). However, for $n > 0$, the average fitting model will depend on the actual location within the time window. This is illustrated in Fig. 9, which shows the normalised a-PPG waveform values at a lag time of +200 ms with respect to the identified $a$ reference points for the 74 cardiac cycles in the above example. The normalised a-PPG waveform values are indicated by line 90. Thus, each value in line 90 corresponds to the value of a normalised a-PPG waveform at lag time +200 ms for one of the cardiac cycles within the time window. For example, the value of line 90 for the 10th cardiac cycle is the value of the normalised a-PPG waveform of the 10th cardiac cycle at +200 ms lag time from the $a$ reference point, the value of line 90 for the 25th cardiac cycle is the value of the normalised a-PPG waveform of the 25th cardiac cycle at +200 ms lag time from the $a$ reference point, and so on. Thus, line 90 in Fig. 9 shows how the values of the normalised a-PPG waveforms at +200ms after the $a$ reference point change within the time window across the cardiac cycles in the PWS.

Fig. 9 also shows the 0-th order average of the normalised a-PPG waveform values at lag time +200 ms (line 92 - which corresponds to the conventional approach to just average (derive the mean) of all values at that lag time), the 1st order average of the normalised a-PPG waveform values at lag time +200 ms (line 94), and the 2nd order average of the normalised a-PPG waveform values at lag time +200 ms (line 96).

Respective versions of Fig. 9 (i.e. respective fitted models) are determined for a range of lag time values. That is, respective versions of Fig. 9 are formed from the values of the normalised a-PPG waveforms at respective lag times. For example, there can be a respective version of Fig. 9 for each of lag times 10 ms, 50 ms, 100 ms, etc. These versions of Fig. 9 will show how the values of the normalised a-PPG waveforms at the respective lag time value change with time across the cardiac cycles in the PWS. For each respective version of Fig. 9, a respective 0th, 1st and/or subsequent order average is determined.

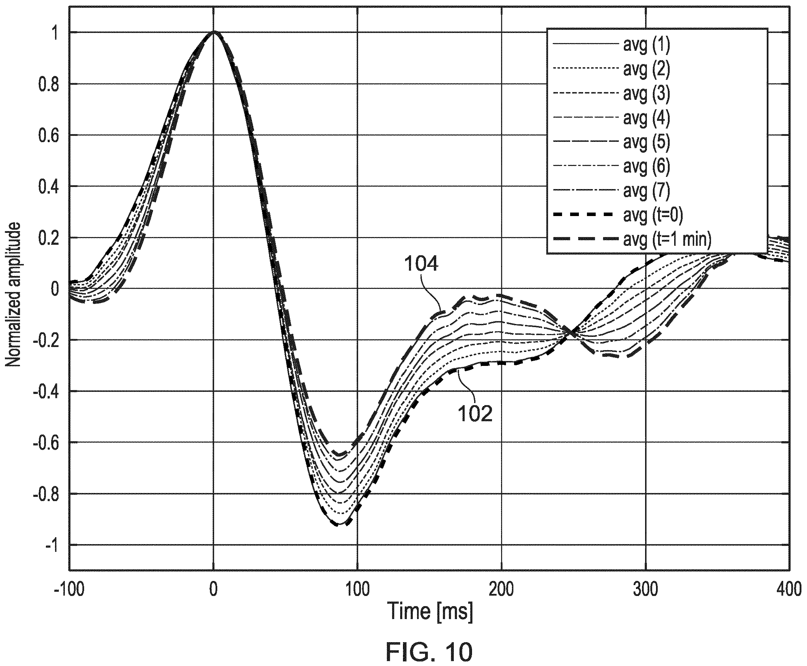

The graph in Fig. 10 shows average normalised cardiac cycle waveforms for several time points $Y$ in the a-PPG signal in Fig. 6(b) using the average (time-varying) fitted models for the range of lag time values ($T_{\Delta k}$) for $n = 1$. Thus, Fig. 10 shows the average waveform resulting from determining the 1st order averages of the normalised a-PPG waveform values for the range of lag time ($T_{\Delta k}$) values between -100 ms and +400 ms. Merely as an example, Fig. 10 shows the average waveform for time point $Y = 0$ sec (labelled 102), time point $Y = 1$ minute (labelled 104), and the average waveforms for the time points corresponding to waveforms 1-7 labelled in Fig. 6(b). Time point $Y = 0$ sec corresponds to the start of the 60-second time period covered by the PWS in Fig. 6, and time point $Y = 60$ sec corresponds to the end of the time period covered by the PWS in Fig. 6.

The average normalised cardiac cycle waveform is derived for the range of lag time values $T_{\Delta k}$, e.g. between -100 ms and +400 ms in the example in Figs. 6-10. The values that form the average normalised cardiac cycle waveform are taken from the set of average (time-varying) fitted models (for a desired value of $n$) for the range of lag time values $T_{\Delta k}$. In other words, for a selected time point $Y$ for which an average normalised cardiac cycle waveform is required, the values that form that average normalised cardiac cycle waveform in the range -100 ms to +400 ms are the values at time point $Y$ in the fitted models (e.g. Fig. 9 line 94, 96) for that range of lag times $T_{\Delta k}$.

As an example, consider Fig. 9 for lag time +200 ms, and consider there to be corresponding versions of Fig. 9 for other lag time values in the range -100 ms to +400 ms. The desired value for $n$ is 1, and the average normalised cardiac cycle waveform (which is shortened to "averaged waveform" herein) is to be derived for time point $Y = 0$ sec (i.e. at the start of the time period covered by the PWS). The value of the average waveform at lag time +200 ms is the value of line 94 in Fig. 9 at time point $Y = 0$ (or at the cardiac cycle index corresponding to $Y = 0$). It can be seen from a comparison of Fig. 9 to the values of the average cardiac cycle waveform in Fig. 10 at a lag value of +200 ms that the value at lag time +200 ms for the averaged waveform for time point $Y = 0$ is -0.3 (i.e. the 1st order polynomial fit 94 has a value of -0.3 for $Y = 0$ (or the cardiac cycle with index 0)), whereas the value at +200 ms for the averaged waveform for time point $Y = 1$ minute is approximately 0 (i.e. the 1st order polynomial fit 94 has an approximate value of 0 for time point $Y = 1$ minute (or the cardiac cycle with index 73)). This is repeated for the other lag time values (i.e. using the other versions of Fig. 9) to derive the full averaged waveform in the range -100 ms to +400 ms. Thus, the value of the averaged waveform for $Y = 0$ at lag time $T_{\Delta k} = -100$ ms is the value of the corresponding line 94 (for lag time $T_{\Delta k} = -100$ ms) at $Y = 0$ (or the cardiac cycle with index 0), the value of the averaged waveform for $Y = 0$ at lag time $T_{\Delta k} = +100$ ms is the value of the corresponding line 94 for lag time $T_{\Delta k} = +100$ ms at $Y = 0$, etc.

The average cardiac cycle waveform for a selected time in the 1-minute time window can therefore be derived from the respective polynomial fit at each of the lag time values. In addition, it can be seen that all intermediate average waveform results (for the times corresponding to the normalised a-PPG waveforms labelled 1 to 7 in Fig. 6(b)) at the different timings within the 1-minute window can also be derived. Therefore, by using an order $n \geq 1$, time-varying changes are accommodated in the averaging procedure. It should be noted that although $n$ can be any integer value equal to or greater than 1, $n = 1$ already gives good results in practice for a PWS representing a cardiac cycle in which the PWS is analysed over a relatively short time period and the underlying changes in the waveform are straightforward. If there are expected to be more substantial changes in the morphology over time, then $n$ can be set to a value higher than 1.

Steps 42, 44 and 46 in Fig. 4 implement the above averaging technique as follows. In line with the above averaging technique, steps 42, 44 and 46 operate on a 'normalised 2PWS'. The normalised 2PWS is obtained by separately normalising each part of the 2PWS corresponding to a respective cardiac cycle identified in step 40 with respect to the amplitude of the 2PWS at the identified reference point for that cardiac cycle. That is, for a particular cardiac cycle identified in step 40, the amplitude of the 2PWS corresponding to that cardiac cycle is normalised around the amplitude of the 2PWS at the identified reference point for that cardiac cycle.

In step 42, for a first lag time value, $T_{\Delta k}$, that is measured with reference to the identified reference points, an n-th order polynomial fit is determined for a first set of values of the normalised 2PWS. As noted above, n is equal to or greater than 1. The first set of values of the normalised 2PWS comprises the values of the 2PWS occurring the first lag time value from the reference point of each identified cardiac cycle. That is, in step 42, for a lag time value of $X$ ms, the first set of values is the values of the normalised 2PWS that are $X$ ms from each of the reference points identified in step 40. In the example shown in Fig. 9, the first set of values corresponds to the line 90. So, for example, for a lag time value $T_{\Delta k}$ of 200 ms, the value for, say, the 20th identified cardiac cycle in Fig. 9 is the value of the normalised 2PWS for the 20th identified cardiac cycle at 200 ms. The line 90 is formed from the values of the normalised 2PWS at 200 ms for each of the identified cardiac cycles. The n-th polynomial fit (with $n \geq 1$) determined in step 42 corresponds to line 94 for $n = 1$ and line 96 for $n = 2$.

In step 44, step 42 is repeated one or more times for one or more further lag time values. Thus, in step 44 one or more further iterations of step 42 are performed for one or more further lag time values to determine respective further n-th order polynomial fits 94, 96 for respective sets of values of the normalised 2PWS. Each of the respective sets of values of the normalised 2PWS comprises the values of the normalised 2PWS that occur the respective further lag time value from the reference point of each identified cardiac cycle. Thus, step 44 results in one or more respective versions of Fig. 9 being derived for the respective lag time value $T_{\Delta k}$.

As noted below with reference to step 46, the number of times that step 42 is repeated determines the time-resolution of the averaged cardiac cycle waveform determined in step 46. The higher the number of times that step 42 is repeated, the higher the smoothness and resolution of the resulting average cardiac cycle waveform. In some embodiments, step 42 can be repeated for lag time values in the range -100 ms to +400 ms. It will be appreciated that an upper limit on the size of the lag time value range can be imposed by the heart rate of the subject (with higher heart rates shortening the range of lag time values, and lower heart rates enabling the range of lag time values to be wider). The lag time value range should cover one cardiac cycle or less (but enough of the cardiac cycle for pulse wave features such as the reflected pulse wave to be observed in the resulting average cardiac cycle waveform).

Next, in step 46, a first average cardiac cycle waveform is formed for a first time point $Y$ in the time period covered by the PWS. For example, for the PPG signal covering the 1-minute time period from which the 2PWS in Fig. 6(b) was derived, the first time point $Y$ could be any of: 0 seconds (i.e. at the start of the 1-minute time period), 25 seconds (i.e. part way through the 1-minute time period), 60 seconds/1 minute (i.e. at the end of the 1-minute time period), etc.

In step 46, rather than evaluate Equation (8) above, the coefficients $a_0(\Delta_k), ..., a_m(\Delta_k)$ are evaluated at a time point $Y$:

$$\psi_{Y,\Delta_k}^n = M_{Y,\Delta_k}^n = a_0 + a_1 \cdot Y \cdot F_s + ... + a_m \cdot (Y \cdot F_s)^n. \qquad (10)$$

where the parameter $F_s$ denotes the sampling-rate of the PWS signal to translate the time point $Y$ into a sample-number, similar to Equation (8).

The first average cardiac cycle waveform is formed from (normalised amplitude) values of the plurality of n-th order polynomial fits 94, 96 at the first time point. Thus, for a time point $Y$ in the time period covered by the PWS, the value of the average cardiac cycle waveform for time point $Y$ is given by the (normalised average) value at time point $Y$ of the $n$-th order polynomial fit 94, 96 for the first set of values determined in step 42 (i.e. the value at time point $Y$ of the n-th order polynomial fit 94, 96 for the first lag time value), and the respective values at time point $Y$ of each of the further n-th order polynomial fits 94, 96 determined in step 44 (i.e. the values at time point $Y$ of the n-th order polynomial fit 94, 96 for the further lag time values).

In a specific example for a time point $Y = 0$ and $n = 1$, the average cardiac cycle waveform is formed from the (normalised amplitude) values at time point $Y$ in the 1st order polynomial fits 94 for each lag time value. Step 46 can result in, for example, an average cardiac cycle waveform 102, 104 as shown in Fig. 10. More generally, step 46 is implemented as described above with reference to Figs. 9 and 10.

In some embodiments, the average cardiac cycle waveform formed in step 46 can be analysed to determine information about a health status of the subject. In some embodiments, the information about the health status is a measurement or indication of the blood pressure of the subject and/or a measurement or indication of a change in the blood pressure of the subject.

In some embodiments, a second average cardiac cycle waveform can be formed for a second time point in the time period covered by the PWS. The second average cardiac cycle waveform can be formed in the same way as the first average cardiac cycle waveform determined in step 46. As an example, one of the first time point and the second time point can be at or near to the start of the PWS, and the other one of the first time point and the second time point can be at or near to the end of the PWS. In further embodiments, one or more further average cardiac cycle waveforms can be determined for respective time points in the time period covered by the PWS.

In embodiments where a second average cardiac cycle waveform is formed, the method can further comprise comparing the first average cardiac cycle waveform and the second average cardiac cycle waveform to determine or identify a change in the average cardiac cycle waveform between the first time point and the second time point. This step can comprise determining a difference signal representing the difference between the two average cardiac cycle waveforms for all lag time values, and/or determining a difference between a specific part or parts of the average cardiac cycle waveforms. For example, a difference can be determined between the magnitude of the minimum in the average cardiac cycle waveforms following the identified $a$ reference point. As another example, a difference can be determined between the timing of different reference points in the two average cardiac cycle waveforms. E.g. reference point e could be identified in each average cardiac cycle waveform, and the time between the respective $a$ and e reference points for each average cardiac cycle waveform can be compared.

In some embodiments, a measure of the blood pressure of the subject, or a measure of a change in blood pressure of the subject can be determined from the first average cardiac cycle waveform and the second average cardiac cycle waveform. In some embodiments, the measure of the blood pressure or change in blood pressure can be determined from the comparison of the first and second average cardiac cycle waveforms. In particular embodiments, a blood pressure surrogate measurement can be determined from the time between the $a$ reference point and the c reference point, which corresponds to the pulse reflection time (PRT).

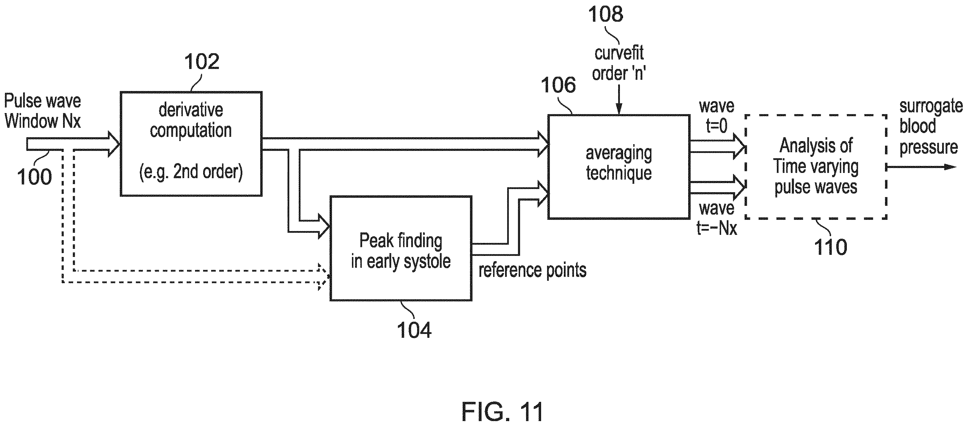

Fig. 11 is a functional block diagram illustrating various operations in analysing a PWS according to various exemplary embodiments. A PWS 100 (e.g. a PPG signal) with a duration $N_x / F_s$ is received by a Derivative Computation block 102 and a Peak Finding block 104. The Derivative Computation block 102 determines the first order derivative of the PWS with respect to time (1PWS) and the second order derivative of the PWS with respect to time (2PWS). The 1PWS and/or 2PWS signals can be provided to the Peak Finding block 104. The Peak Finding block 104 evaluates the input signals to identify cardiac cycles and the reference points corresponding to a part of the cardiac cycle in the 2PWS (e.g. the early systole/up-flank $a$ reference point). The operations of the Derivative Computation block 102 and the Peak Finding block 104 correspond generally to step 40 described above.

The 2PWS and the identified reference points are input to an Averaging block 106. A desired value 108 of $n$ for the polynomial fit is input to the Averaging block 106. Alternatively the desired value of $n$ can be predetermined or preset in the Averaging block 106. The Averaging block 106 implements the waveform averaging process described above in steps 42, 44 and 46 based on the input 2PWS and the identified reference points. The Averaging block 106 outputs at least one average cardiac cycle waveform for a particular time point. In Fig. 11 the Averaging block 106 is shown as outputting an average cardiac cycle waveform for $Y = 0$ and $Y = -N_x / F_s$ (e.g. 1 minute prior to t = 0).

In optional embodiments, the average cardiac cycle waveform can be input to an Analysis block 110 that can perform some analysis of the average cardiac cycle waveform to determine information about the health status of the subject. In some embodiments, the Analysis block 110 can determine a surrogate blood pressure measurement. As an example, the Analysis block 110 could subtract the two averaged cardiac cycle waveforms as follows:

$$\Delta \psi^n_{\Delta_k} = \psi^n_{Y=60,\Delta_k} - \psi^n_{Y=0,\Delta_k}, \tag{11}$$

where: $\psi^n_{Y=0,\Delta_k}$ is the first average cardiac cycle waveform for a time point $Y = 0$ and $\psi^n_{Y=60,\Delta_k}$ is the second average cardiac cycle waveform for a time point $Y = 60$ seconds.

The value of $\Delta \psi^n_{\Delta_k}$ is indicative of the changes of energy in the pulse wave in the course of the window length (e.g. 1 minute) at a specific lag-time $T_{\Delta k}$. Since blood pressure changes will lead to changes of the energy in the pulse wave at lag times where reflections are to be expected (e.g. reflections from renal bifurcations), the value of $\Delta \psi^n_{\Delta_k}$ can be exploited to estimate the amount of blood pressure change in the course of the window length.

Therefore there is provided techniques for the improved averaging of cardiac cycle waveforms.

## Claims

1. A computer-implemented method for analysing a pulse wave signal (PWS), obtained from a subject, wherein the pulse wave signal (PWS) comprises pulse wave measurements for a plurality of cardiac cycles of the subject during a first time period, the method comprising:

   (i) analysing (40) the pulse wave signal (PWS) to identify a plurality of cardiac cycles and a respective reference point for each identified cardiac cycle;

   **characterized in that** the method further comprises:

   (ii) determining a second derivative with respect to time of the pulse wave signal (PWS);
   (iii) determining a normalised second derivative by, for each part of the second derivative corresponding to a respective identified cardiac cycle, normalising said part of the second derivative with respect to the amplitude of the second derivative at the identified reference point for said cardiac cycle;
   (iv) for a first lag time value, determining (42) an $n$-th order polynomial fit for a first set of values of the normalised second derivative, wherein the first set of values of the normalised second derivative comprises the values of the normalised second derivative occurring the first lag time value from the reference point of each identified cardiac cycle, wherein $n$ is equal to or greater than 1;
   (v) performing (44) one or more further iterations of step (iv) for one or more further lag time values to determine respective further $n$-th order polynomial fits for respective sets of values of the normalised second derivative, wherein a respective set of values of the normalised second derivative comprises the values of the normalised second derivative that occur the respective further lag time value from the reference point of each identified cardiac cycle; and
   (vi) forming (46) a first average cardiac cycle waveform for a first time point in the first time period, wherein the first average cardiac cycle waveform is formed from values of the plurality of n-th order polynomial fits at the first time point.

2. A method as claimed in claim 1, wherein the method further comprises:
   forming a second average cardiac cycle waveform for a second time point in the first time period, wherein the second average cardiac cycle waveform is formed from values of the plurality of n-th order polynomial fits at the second time point.

3. A method as claimed in claim 2, wherein the method further comprises:
   comparing the first average cardiac cycle waveform and the second average cardiac cycle waveform to determine a change in the average cardiac cycle waveform between the first time point and the second time point.

4. A method as claimed in claim 2 or 3, wherein the method further comprises:
   determining a measure of the blood pressure of the subject, or a measure of a change in blood pressure of the subject, from the first average cardiac cycle waveform and the second average cardiac cycle waveform.

5. A method as claimed in claim 1, wherein the method further comprises:
processing the first average cardiac cycle waveform to determine a measure of the blood pressure of the subject.

6. A method as claimed in any of claims 1-5, wherein each of the first lag time value and the one or more further lag time values are equal to or less than a duration of a cardiac cycle of the subject.

7. A method as claimed in any of claims 1-6, wherein the reference point for each identified cardiac cycle is an onset of a pulse wave of the subject.

8. An apparatus (30) for analysing a pulse wave signal (PWS), obtained from a subject, wherein the pulse wave signal (PWS) comprises pulse wave measurements for a plurality of cardiac cycles of the subject during a first time period, the apparatus (30) configured to:

   (i) analyse the pulse wave signal (PWS) to identify a plurality of cardiac cycles and a respective reference point for each identified cardiac cycle;

   **characterized in that** the apparatus further comprises:

   (ii) determine a second derivative with respect to time of the pulse wave signal (PWS);
   (iii) determine a normalised second derivative by, for each part of the second derivative corresponding to a respective identified cardiac cycle, normalising said part of the second derivative with respect to the amplitude of the second derivative at the identified reference point for said cardiac cycle;
   (iv) for a first lag time value, determine an $n$-th order polynomial fit for a first set of values of the normalised second derivative, wherein the first set of values of the normalised second derivative comprises the values of the normalised second derivative occurring the first lag time value from the reference point of each identified cardiac cycle, wherein $n$ is equal to or greater than 1;
   (v) perform one or more further iterations of operation (iv) for one or more further lag time values to determine respective further $n$-th order polynomial fits for respective sets of values of the normalised second derivative, wherein a respective set of values of the normalised second derivative comprises the values of the normalised second derivative that occur the respective further lag time value from the reference point of each identified cardiac cycle; and
   (vi) form a first average cardiac cycle waveform for a first time point in the first time period, wherein the first average cardiac cycle waveform is formed from values of the plurality of n-th order polynomial fits at the first time point.

9. An apparatus (30) as claimed in claim 8, wherein the apparatus (30) is further configured to:
form a second average cardiac cycle waveform for a second time point in the first time period, wherein the second average cardiac cycle waveform is formed from values of the plurality of n-th order polynomial fits at the second time point.

10. An apparatus (30) as claimed in claim 9, wherein the apparatus (30) is further configured to:
compare the first average cardiac cycle waveform and the second average cardiac cycle waveform to determine a change in the average cardiac cycle waveform between the first time point and the second time point.

11. An apparatus (30) as claimed in claim 9 or 10, wherein the apparatus (30) is further configured to:
determine a measure of the blood pressure of the subject, or a measure of a change in blood pressure of the subject, from the first average cardiac cycle waveform and the second average cardiac cycle waveform.

12. An apparatus (30) as claimed in claim 8, wherein the apparatus (30) is further configured to:
process the first average cardiac cycle waveform to determine a measure of the blood pressure of the subject.

13. An apparatus (30) as claimed in any of claims 8-12, wherein each of the first time lag value and the one or more further lag time values are equal to or less than a duration of a cardiac cycle of the subject.

14. An apparatus (30) as claimed in any of claims 8-13, wherein the reference point for each identified cardiac cycle is an onset of a pulse wave of the subject.

15. A computer program product comprising computer readable code configured such that, on execution by a suitable computer or processor, the computer or processor is caused to perform the method of any of claims 1-7.

**Patentansprüche**

1. Computerimplementiertes Verfahren zum Analysieren eines Pulswellensignals (PWS), das von einem Subjekt erhalten wurde, wobei das Pulswellensignal (PWS) Pulswellenmessungen für eine Vielzahl von Herzzyklen des Subjekts während eines ersten Zeitraums umfasst, wobei das Verfahren umfasst:

   (i) Analysieren (40) des Pulswellensignals (PWS), um eine Vielzahl von Herzzyklen und einen jeweiligen Referenzpunkt für jeden identifizierten Herzzyklus zu identifizieren;

   **dadurch gekennzeichnet, dass** das Verfahren weiter umfasst:

   (ii) Bestimmen einer zweiten Ableitung in Bezug auf Zeit des Pulswellensignals (PWS);
   (iii) Bestimmen einer normalisierten zweiten Ableitung durch Normalisieren des Teils der zweiten Ableitung in Bezug auf die Amplitude der zweiten Ableitung am identifizierten Referenzpunkt für den Herzzyklus für jeden Teil der zweiten Ableitung, der einem jeweiligen identifizierten Herzzyklus entspricht;
   (iv) für einen ersten Verzögerungszeitwert Bestimmen (42) einer Polynomanpassung n-ter Ordnung für einen ersten Satz von Werten der normalisierten zweiten Ableitung, wobei der erste Satz von Werten der normalisierten zweiten Ableitung die Werte der normalisierten zweiten Ableitung umfasst, die beim ersten Verzögerungszeitwert vom Referenzpunkt jedes identifizierten Herzzyklus auftreten, wobei n gleich oder größer ist als 1;
   (v) Durchführen (44) einer oder mehrerer weiterer Iterationen von Schritt (iv) für einen oder mehrere weitere Verzögerungszeitwerte, um jeweilige weitere Polynomanpassungen n-ter Ordnung für jeweilige Wertesätze der normalisierten zweiten Ableitung zu bestimmen, wobei ein jeweiliger Wertesatz der normalisierten zweiten Ableitung die Werte der normalisierten zweiten Ableitung umfasst, die beim jeweiligen weiteren Verzögerungszeitwert vom Referenzpunkt jedes identifizierten Herzzyklus auftreten; und
   (vi) Bilden (46) einer ersten durchschnittlichen Herzzykluswellenform für einen ersten Zeitpunkt in dem ersten Zeitraum, wobei die erste durchschnittliche Herzzykluswellenform aus Werten der Vielzahl von Polynomanpassungen n-ter Ordnung zu dem ersten Zeitpunkt gebildet wird.

2. Verfahren nach Anspruch 1, wobei das Verfahren weiter umfasst:
   Bilden einer zweiten durchschnittlichen Herzzykluswellenform für einen zweiten Zeitpunkt in dem ersten Zeitraum, wobei die zweite durchschnittliche Herzzykluswellenform aus Werten der Vielzahl von Polynomanpassungen n-ter Ordnung zu dem zweiten Zeitpunkt gebildet wird.

3. Verfahren nach Anspruch 2, wobei das Verfahren weiter umfasst:
   Vergleichen der ersten durchschnittlichen Herzzykluswellenform und der zweiten durchschnittlichen Herzzykluswellenform, um eine Änderung in der durchschnittlichen Herzzykluswellenform zwischen dem ersten Zeitpunkt und dem zweiten Zeitpunkt zu bestimmen.

4. Verfahren nach einem der Ansprüche 2 oder 3, wobei das Verfahren weiter umfasst:
   Bestimmen eines Maßes für den Blutdruck des Subjekts oder eines Maßes für eine Änderung im Blutdruck des Subjekts aus der ersten durchschnittlichen Herzzykluswellenform und der zweiten durchschnittlichen Herzzykluswellenform.

5. Verfahren nach Anspruch 1, wobei das Verfahren weiter umfasst:
   Verarbeiten der ersten durchschnittlichen Herzzykluswellenform, um ein Maß für den Blutdruck des Subjekts zu bestimmen.

6. Verfahren nach einem der Ansprüche 1-5, wobei jeder von dem ersten Verzögerungszeitwert und dem einen oder den mehreren weiteren Verzögerungszeitwerten jeweils gleich oder kleiner als eine Dauer eines Herzzyklus des Subjekts ist.

7. Verfahren nach einem der Ansprüche 1-6, wobei der Referenzpunkt für jeden identifizierten Herzzyklus ein Beginn einer Pulswelle des Subjekts ist.

8. Einrichtung (30) zum Analysieren eines Pulswellensignals (PWS), das von einem Subjekt erhalten wurde, wobei das Pulswellensignal (PWS) Pulswellenmessungen für eine Vielzahl von Herzzyklen des Subjekts während eines ersten Zeitraums umfasst, wobei die Einrichtung (30) konfiguriert ist zum:

(i) Analysieren des Pulswellensignals (PWS), um eine Vielzahl von Herzzyklen und einen jeweiligen Referenzpunkt für jeden identifizierten Herzzyklus zu identifizieren;

**dadurch gekennzeichnet, dass** die Einrichtung weiter Folgendes umfasst:

(ii) Bestimmen einer zweiten Ableitung in Bezug auf Zeit des Pulswellensignals (PWS);
(iii) Bestimmen einer normalisierten zweiten Ableitung durch Normalisieren des Teils der zweiten Ableitung in Bezug auf die Amplitude der zweiten Ableitung am identifizierten Referenzpunkt für den Herzzyklus für jeden Teil der zweiten Ableitung, der einem jeweiligen identifizierten Herzzyklus entspricht;
(iv) für einen ersten Verzögerungszeitwert, Bestimmen einer Polynomanpassung n-ter Ordnung für einen ersten Satz von Werten der normalisierten zweiten Ableitung, wobei der erste Satz von Werten der normalisierten zweiten Ableitung die Werte der normalisierten zweiten Ableitung umfasst, die beim ersten Verzögerungszeitwert vom Referenzpunkt jedes identifizierten Herzzyklus auftreten, wobei n gleich oder größer ist als 1;
(v) Durchführen einer oder mehrerer weiterer Iterationen von Vorgang (iv) für einen oder mehrere weitere Verzögerungszeitwerte, um jeweilige weitere Polynomanpassungen n-ter Ordnung für jeweilige Wertesätze der normalisierten zweiten Ableitung zu bestimmen, wobei ein jeweiliger Wertesatz der normalisierten zweiten Ableitung die Werte der normalisierten zweiten Ableitung umfasst, die beim jeweiligen weiteren Verzögerungszeitwert vom Referenzpunkt jedes identifizierten Herzzyklus auftreten; und
(vi) Bilden einer ersten durchschnittlichen Herzzykluswellenform für einen ersten Zeitpunkt in dem ersten Zeitraum, wobei die erste durchschnittliche Herzzykluswellenform aus Werten der Vielzahl von Polynomanpassungen n-ter Ordnung zum ersten Zeitpunkt gebildet wird.

9. Einrichtung (30) nach Anspruch 8, wobei die Einrichtung (30) weiter konfiguriert ist zum:
Bilden einer zweiten durchschnittlichen Herzzykluswellenform für einen zweiten Zeitpunkt in dem ersten Zeitraum, wobei die zweite durchschnittliche Herzzykluswellenform aus Werten der Vielzahl von Polynomanpassungen n-ter Ordnung zu dem zweiten Zeitpunkt gebildet wird.

10. Einrichtung (30) nach Anspruch 9, wobei die Einrichtung (30) weiter konfiguriert ist zum:
Vergleichen der ersten durchschnittlichen Herzzykluswellenform und der zweiten durchschnittlichen Herzzykluswellenform, um eine Änderung in der durchschnittlichen Herzzykluswellenform zwischen dem ersten Zeitpunkt und dem zweiten Zeitpunkt zu bestimmen.

11. Einrichtung (30) nach Anspruch 9 oder 10, wobei die Einrichtung (30) weiter konfiguriert ist zum:
Bestimmen eines Maßes für den Blutdruck des Subjekts oder eines Maßes für eine Änderung im Blutdruck des Subjekts aus der ersten durchschnittlichen Herzzykluswellenform und der zweiten durchschnittlichen Herzzykluswellenform.

12. Einrichtung (30) nach Anspruch 8, wobei die Einrichtung (30) weiter konfiguriert ist zum:
Verarbeiten der ersten durchschnittlichen Herzzykluswellenform, um ein Maß für den Blutdruck des Subjekts zu bestimmen.

13. Einrichtung (30) nach einem der Ansprüche 8-12, wobei jeder von dem ersten Zeitverzögerungswert und dem einen oder den mehreren weiteren Verzögerungszeitwerten jeweils gleich oder kleiner als eine Dauer eines Herzzyklus des Subjekts ist.

14. Einrichtung (30) nach einem der Ansprüche 8-13, wobei der Referenzpunkt für jeden identifizierten Herzzyklus ein Beginn einer Pulswelle des Subjekts ist.

15. Computerprogrammprodukt, das computerlesbaren Code umfasst, der dazu konfiguriert ist, bei Ausführung durch einen geeigneten Computer oder Prozessor den Computer oder Prozessor zu veranlassen, das Verfahren nach einem der Ansprüche 1-7 durchzuführen.

**Revendications**

1. Procédé mis en œuvre par ordinateur pour analyser un signal d'onde d'impulsion (PWS), obtenu d'un sujet, dans lequel le signal d'onde d'impulsion (PWS) comprend des mesures d'onde d'impulsion pour une pluralité de cycles cardiaques du sujet pendant une première période de temps, le procédé comprenant :

(i) l'analyse (40) du signal d'onde d'impulsion (PWS) pour identifier une pluralité de cycles cardiaques et un point de référence respectif pour chaque cycle cardiaque identifié ;

**caractérisé en ce que** le procédé comprend en outre :

(ii) la détermination d'une seconde dérivée par rapport au temps du signal d'onde d'impulsion (PWS) ;

(iii) la détermination d'une seconde dérivée normalisée en normalisant, pour chaque partie de la seconde dérivée correspondant à un cycle cardiaque identifié respectif, ladite partie de la seconde dérivée par rapport à l'amplitude de la seconde dérivée au point de référence identifié pour ledit cycle cardiaque ;

(iv) pour une première valeur de temps de décalage, la détermination (42) d'un ajustement polynomial d'ordre n pour un premier ensemble de valeurs de la seconde dérivée normalisée, dans lequel le premier ensemble de valeurs de la seconde dérivée normalisée comprend les valeurs de la seconde dérivée normalisée se produisant à la première valeur de temps de décalage à partir du point de référence de chaque cycle cardiaque identifié, dans lequel n est égal ou supérieur à 1 ;

(v) la réalisation (44) d'une ou de plusieurs itérations supplémentaires de l'étape (iv) pour une ou plusieurs autres valeurs de temps de décalage pour déterminer d'autres ajustements polynomiaux d'ordre n respectifs pour des ensembles respectifs de valeurs de la seconde dérivée normalisée, dans lequel un ensemble respectif de valeurs de la seconde dérivée normalisée comprend les valeurs de la seconde dérivée normalisée qui se produisent à la valeur de temps de décalage supplémentaire respective à partir du point de référence de chaque cycle cardiaque identifié ; et

(vi) la formation (46) d'une première forme d'onde de cycle cardiaque moyenne pour un premier instant pendant la première période de temps, dans lequel la première forme d'onde de cycle cardiaque moyenne est formée à partir de valeurs de la pluralité d'ajustements polynomiaux d'ordre n au premier instant.

2.  Procédé selon la revendication 1, dans lequel le procédé comprend en outre :
    la formation d'une seconde forme d'onde de cycle cardiaque moyenne pour un second instant pendant la première période de temps, dans lequel la seconde forme d'onde de cycle cardiaque moyenne est formée à partir de valeurs de la pluralité d'ajustements polynomiaux d'ordre n au second instant.

3.  Procédé selon la revendication 2, dans lequel le procédé comprend en outre :
    la comparaison de la première forme d'onde de cycle cardiaque moyenne et de la seconde forme d'onde de cycle cardiaque moyenne pour déterminer un changement dans la forme d'onde de cycle cardiaque moyenne entre le premier instant et le second instant.

4.  Procédé selon la revendication 2 ou 3, dans lequel le procédé comprend en outre :
    la détermination d'une mesure de la pression artérielle du sujet, ou d'une mesure d'un changement de la pression artérielle du sujet, à partir de la première forme d'onde de cycle cardiaque moyenne et de la seconde forme d'onde de cycle cardiaque moyenne.

5.  Procédé selon la revendication 1, dans lequel le procédé comprend en outre :
    le traitement de la première forme d'onde de cycle cardiaque moyenne pour déterminer une mesure de la pression artérielle du sujet.

6.  Procédé selon l'une quelconque des revendications 1-5, dans lequel chacune de la première valeur de temps de décalage et des une ou plusieurs valeurs de temps de décalage supplémentaires sont égales ou inférieures à une durée d'un cycle cardiaque du sujet.

7.  Procédé selon l'une quelconque des revendications 1-6, dans lequel le point de référence pour chaque cycle cardiaque identifié est un début d'une onde d'impulsion du sujet.

8.  Appareil (30) pour analyser un signal d'onde d'impulsion (PWS), obtenu d'un sujet, dans lequel le signal d'onde d'impulsion (PWS) comprend des mesures d'onde d'impulsion pour une pluralité de cycles cardiaques du sujet pendant une première période de temps, l'appareil (30) étant configuré pour :

    (i) analyser le signal d'onde d'impulsion (PWS) pour identifier une pluralité de cycles cardiaques et un point de référence respectif pour chaque cycle cardiaque identifié ;

    **caractérisé en ce que** l'appareil comprend en outre :

(ii) la détermination d'une seconde dérivée par rapport au temps du signal d'onde d'impulsion (PWS) ;

(iii) la détermination d'une seconde dérivée normalisée en normalisant, pour chaque partie de la seconde dérivée correspondant à un cycle cardiaque identifié respectif, ladite partie de la seconde dérivée par rapport à l'amplitude de la seconde dérivée au point de référence identifié pour ledit cycle cardiaque ;

(iv) pour une première valeur de temps de décalage, la détermination d'un ajustement polynomial d'ordre n pour un premier ensemble de valeurs de la seconde dérivée normalisée, dans lequel le premier ensemble de valeurs de la seconde dérivée normalisée comprend les valeurs de la seconde dérivée normalisée se produisant à la première valeur de temps de décalage à partir du point de référence de chaque cycle cardiaque identifié, dans lequel n est égal ou supérieur à 1 ;

(v) la réalisation d'une ou de plusieurs itérations supplémentaires de l'opération (iv) pour une ou plusieurs valeurs de temps de décalage supplémentaires pour déterminer des ajustements polynomiaux d'ordre n supplémentaires respectifs pour des ensembles respectifs de valeurs de la seconde dérivée normalisée, dans lequel un ensemble respectif de valeurs de la seconde dérivée normalisée comprend les valeurs de la seconde dérivée normalisée qui se produisent à la valeur de temps de décalage supplémentaire respective à partir du point de référence de chaque cycle cardiaque identifié ; et

(vi) la formation d'une première forme d'onde de cycle cardiaque moyenne pour un premier instant pendant la première période de temps, dans lequel la première forme d'onde de cycle cardiaque moyenne est formée à partir de valeurs de la pluralité d'ajustements polynomiaux d'ordre n au premier instant.

9. Appareil (30) selon la revendication 8, dans lequel l'appareil (30) est en outre configuré pour :
former une seconde forme d'onde de cycle cardiaque moyenne pour un second instant pendant la première période de temps, dans lequel la seconde forme d'onde de cycle cardiaque moyenne est formée à partir de valeurs de la pluralité d'ajustements polynomiaux d'ordre n au second instant.

10. Appareil (30) selon la revendication 9, dans lequel l'appareil (30) est en outre configuré pour :
comparer la première forme d'onde de cycle cardiaque moyenne et la seconde forme d'onde de cycle cardiaque moyenne pour déterminer un changement dans la forme d'onde de cycle cardiaque moyenne entre le premier instant et le second instant.

11. Appareil (30) selon la revendication 9 ou 10, dans lequel l'appareil (30) est en outre configuré pour :
déterminer une mesure de la pression artérielle du sujet, ou une mesure d'un changement de la pression artérielle du sujet, à partir de la première forme d'onde de cycle cardiaque moyenne et de la seconde forme d'onde de cycle cardiaque moyenne.

12. Appareil (30) selon la revendication 8, dans lequel l'appareil (30) est en outre configuré pour :
traiter la première forme d'onde de cycle cardiaque moyenne pour déterminer une mesure de la pression artérielle du sujet.

13. Appareil (30) selon l'une quelconque des revendications 8-12, dans lequel chacune de la première valeur de décalage temporel et des une ou plusieurs valeurs de temps de décalage supplémentaires sont égales ou inférieures à une durée d'un cycle cardiaque du sujet.

14. Appareil (30) selon l'une quelconque des revendications 8-13, dans lequel le point de référence pour chaque cycle cardiaque identifié est un début d'une onde d'impulsion du sujet.

15. Produit de programme informatique comprenant un code lisible par ordinateur configuré de telle sorte que, lors de son exécution par un ordinateur ou un processeur approprié, l'ordinateur ou le processeur soit amené à réaliser le procédé selon l'une quelconque des revendications 1-7.

FIG. 1

EP 4 262 526 B1

(a)

MAP [mmHg]

(b)

PWV [m/s]

FIG. 2

EP 4 262 526 B1

30

34 — Processing unit

32

Pulse wave sensor

36 — Memory unit

38 — User Interface

FIG. 3

Analyse PWS to identify a plurality of cardiac cycles and a reference point for each cycle — 40

For a first lag time value, determine an n-th order polynomial fit for a first set of values of a normalised second derivative with respect to time of the PWS — 42

Perform one or more further iterations of step 42 for one or more further lag time values — 44

Form a first average cardiac cycle waveform for a first time point in the first time period — 46

FIG. 4

FIG. 5

FIG. 6

FIG. 7

EP 4 262 526 B1

FIG. 8

FIG. 9

FIG. 10

FIG. 11

Pulse wave
Window Nx

100

derivative
computation
(e.g. 2nd order)

102

Peak finding
in early systole

104

reference points

averaging
technique

106

curvefit
order 'n'

108

wave
t=0

wave
t=-Nx

Analysis of
Time varying
pulse waves

110

surrogate
blood
pressure

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016228069 A1
- AU 2019260099 A1

- WO 2015044010 A

**Non-patent literature cited in the description**

- **HANGSIK SHIN et al.** Feasibility study for the non-invasive blood pressure estimation based on ppg morphology: normotensive subject study. *Biomed. Eng*, 2017, vol. 16, 10